# EUROPÄISCHE PATENTSCHRIFT

(11) **EP 2 063 937 B1**
(45) Veröffentlichungstag und Bekanntmachung des Hinweises auf die Patenterteilung: **03.07.2019**
(21) Anmeldenummer: 07785108.7
(22) Anmeldetag: 22.08.2007
(51) Int. Cl.: A61M 5/32

(54) **NADELSCHUTZVORRICHTUNG MIT BLOCKIERTER SCHUTZPOSITION**
NEEDLE PROTECTION DEVICE WITH A BLOCKED PROTECTIVE POSITION
DISPOSITIF DE PROTECTION D'AIGUILLE PRÉSENTANT UNE POSITION DE PROTECTION BLOQUÉE

(30) Priorität: 06.09.2006 DE 102006042236
(43) Veröffentlichungstag der Anmeldung: 03.06.2009
(73) Patentinhaber: TecPharma Licensing AG, 3400 Burgdorf (CH)
(72) Erfinder: GRATWOHL, Christian, 5000 Aarau (CH); WYMANN, Martin, 3097 Liebefeld (CH)
(86) Internationale Anmeldenummer: PCT/CH2007/000413
(87) Internationale Veröffentlichungsnummer: WO 2008/028304

(56) Entgegenhaltungen:
- EP-A- 1 464 353
- EP-A- 1 557 191
- WO-A-01/91837
- WO-A-02/20074
- WO-A-2004/000397
- FR-A- 2 881 053

## Beschreibung

Die Erfindung betrifft eine Nadelschutzvorrichtung, die an einem Injektionsgerät befestigt oder befestigbar ist. Die Nadelschutzvorrichtung findet bevorzugt in der Verabreichung, beispielsweise Selbstverabreichung von Medikamenten Verwendung, beispielsweise der Verabreichung von Insulin, und kann insbesondere in der Selbstverabreichung verwendet werden, d.h. von Patienten, die sich das betreffende Medikament selbst verabreichen. Das Injektionsgerät kann eine einfache Spritze sein, auch eine Spritze, die nach einmaligem Gebrauch entsorgt wird. In derartigen Verwendungen kann die Nadelschutzvorrichtung entweder separat von dem Injektionsgerät hergestellt und für die Verabreichung mit dem Injektionsgerät verbunden werden oder gleich integrierter Bestandteil des Injektionsgeräts sein und gemeinsam mit diesem entsorgt werden. Bevorzugter handelt es sich bei dem Injektionsgerät jedoch um eines für wiederholten Gebrauch, und besonders bevorzugt handelt es sich um ein Injektionsgerät, das die Einstellung der Dosis des zu verabreichenden Produkts zulässt. Das Injektionsgerät kann insbesondere ein Injektionspen sein, wie er beispielsweise in der Diabetestherapie und mittlerweile auch anderen Therapien verwendet wird.

Bei der Handhabung von Injektionsgeräten besteht die Gefahr, dass Patienten oder medizinisches Fachpersonal sich an spitzen Injektionsnadeln verletzen und an einer bereits einmal benutzten Injektionsnadel infizieren. Um dies zu verhindern, wurden Nadelschutzvorrichtungen mit beweglichem Nadelschutz entwickelt. Für die Injektion wird das mit der Nadelschutzvorrichtung ausgestattete Injektionsgerät mit dem Nadelschutz gegen die Haut gedrückt. Das Injektionsgerät mit der davon in die distale Richtung vorragenden Injektionsnadel bewegt sich aufgrund des Drucks relativ zu dem Nadelschutz in die distale Richtung, so dass die Injektionsnadel die Haut durchsticht.

Der Nadelschutz vollführt relativ zu dem Injektionsgerät entsprechend eine Bewegung in die proximale Richtung bis in eine Freigabeposition, in der die Injektionsnadel mit ihrem in die Haut eindringenden Injektionsabschnitt in distaler Richtung über den Nadelschutz vorsteht. Wird die Injektionsnadel wieder aus der Haut gezogen, bewegt sich der Nadelschutz aufgrund der Beaufschlagung mit einer Elastizitätskraft eines Rückstellglieds wieder in die distale Richtung, bis in eine Schutzposition, in der er die Injektionsnadel einschließlich einer Nadelspitze überragt. Mit Erreichen der Schutzposition verriegelt sich der Nadelschutz selbsttätig, so dass er sich relativ zu der Injektionsnadel nicht wieder in die proximale Richtung zurückbewegen kann.

Aus der US 6,773,415 B2 ist eine derartige Nadelschutzvorrichtung bekannt, die eine Injektionsnadel, einen Nadelhalter und einen Nadelschutz umfasst. Der Nadelschutz umgibt den Nadelhalter und ist an diesem axial beweglich gelagert. In dem Nadelschutz ist ein Blockierglied axial beweglich aufgenommen, das sich in der Freigabeposition des Nadelschutzes an dem Nadelhalter und bei dem Herausziehen der Injektionsnadel an dem Nadelschutz blockiert, so dass nach der Injektion der Nadelschutz über das Blockierglied relativ zu dem Nadelhalter axial fixiert ist. Da das Blockierglied sowohl an dem Nadelhalter als auch an dem Nadelschutz axial blockiert und ferner im Gleitkontakt mit dem Nadelschutz eine saubere axiale Führung gewährleistet werden muss, weist es eine komplexe Form auf. Ferner bedarf der Nadelhalter einer besonderen Formgebung, um einerseits den Nadelschutz zu führen und zu halten und andererseits die axiale Blockierung des Blockierglieds zu ermöglichen. Das Rückstellglied muss ferner axial durch das Blockierglied geführt werden und muss deshalb vergleichsweise schlank sein.

Eine Nadelschutzvorrichtung, die einschließlich Injektionsnadel an einem Injektionsgerät befestigbar ist, beschreibt beispielsweise die WO 01/91837 A1. Die Vorrichtung umfasst einen Nadelhalter mit einer in die distale Richtung vorragenden Injektionsnadel und einen Nadelschutz, der an dem Nadelhalter so gelagert ist, dass er aus einer proximalen Position gegen die Kraft eines Rückstellglieds bis in die Freigabeposition und aus der Freigabeposition zurück in die distale Position bewegbar ist. Der Nadelhalter bildet eine verzweigte Führungsbahn und der Nadelschutz ein längs der Führungsbahn geführtes Eingriffselement. Das Eingriffselement durchläuft bei einer Injektion nacheinander die beiden Zweige der Führungsbahn und verrastet nach dem Herausziehen der Injektionsnadel am Ende des einen Zweigs der Führungsbahn, so dass der Nadelschutz gegen eine erneute Bewegung in die proximale Richtung blockiert ist. Auch diese Nadelschutzvorrichtung erfordert eine komplexe Form des Nadelhalters. Zudem ist nicht mit der wünschenswerten Sicherheit gewährleistet, dass sich der die Schutzposition bestimmende Rasteingriff nicht versehentlich wieder löst, da das Eingriffselement vor dem Verrasten mehrmals elastisch gebogen wird und deshalb nach außen abstehen kann.

Ferner ist in der EP1557191 A2 eine Sicherheitsnadel offenbart, welche ein Verriegelungselement aufweist, welches zwischen einer Feder und einer Schutzvorrichtung angeordnet ist und gleichzeitig mit der Schutzvorrichtung relativ zu einem Gehäuse der Sicherheitsnadel während der Benutzung in Längsrichtung bewegbar ist.

Es ist eine Aufgabe der Erfindung, eine Nadelschutzvorrichtung zu schaffen, die einfach herstellbar ist und den Verwender nach einer Injektion sicher vor Kontakt mit der Injektionsnadel schützt.

Die Erfindung geht von einer Nadelschutzvorrichtung aus, die an einem Injektionsgerät befestigt oder für eine Befestigung vorgesehen ist und für die Befestigung einen Nadelhalter mit einer in eine distale Richtung vorragenden Injektionsnadel aufweist. Die Nadelschutzvorrichtung umfasst ferner eine Blockiereinrichtung, die von dem Nadelhalter ebenfalls in die distale Richtung vorragt und radial von der Injektionsnadel beabstandet ist, wobei sich die Angabe radial auf die Achse distal-proximal bezieht. Die Blockiereinrichtung ist oder umfasst vorzugsweise ein Hohlprofil, das die Injektionsnadel über einen Längenabschnitt umlaufend umgibt. Bestandteil der Nadelschutzvorrichtung ist ferner ein Nadelschutz, der radial zwischen der Injektionsnadel und der Blockiereinrichtung in die proximale Richtung bis in eine die Injektionsnadel freigebende Freigabeposition und aus der Freigabeposition in die distale Richtung bis in eine Schutzposition bewegbar ist, in der er die Injektionsnadel einschließlich einer Nadelspitze umgibt. Die Blockiereinrichtung und der Nadelschutz wirken auf eine Weise zusammen, die sicherstellt, dass der Nadelschutz in der Schutzposition gegen eine erneute Bewegung in die proximale Richtung blockierbar ist, d.h. bei Erreichen der Schutzposition in dieser blockiert wird. Der Nadelschutz umgibt die Injektionsnadel, so dass in der Schutzposition ein Zugriff auf die Injektionsnadel sicher verhindert wird. Bevorzugt ist der Nadelschutz eine zumindest im Wesentlichen umlaufend zumindest im Wesentlichen geschlossene Hülse. Grundsätzlich kann er jedoch auch größere Durchbrechungen aüfweisen, solange er in der Schutzposition nur seine Schutzfunktion erfüllt, d.h. den Verwender vor Stichverletzungen schützt.

Nach der Erfindung umfasst die Nadelschutzvorrichtung zusätzlich ein Sicherungsglied, das radial zwischen der Injektionsnadel und dem Nadelschutz angeordnet und relativ zu der Injektionsnadel und der Blockiereinrichtung aus einer proximalen Position, die es in einem Ausgangszustand vor der Injektion einnimmt, in die distale Richtung bewegbar ist. Die Bewegung in die distale Richtung wird durch den Nadelschutz bewirkt, wenn dieser sich nach der Injektion in die Schutzposition bewegt. Der Nadelschutz nimmt das Sicherungsglied in die Schutzposition mit. Um die Mitnahme zu bewirken, weisen der Nadelschutz ein Eingriffselement und das Sicherungsglied ein Eingriffsgegenelement auf, die miteinander in einen Mitnahmeeingriff gelangen, wenn der Nadelschutz in die Freigabeposition bewegt wird.

In einer ersten Ausführungsform gelangen das Eingriffselement und das Eingriffsgegenelement genau in dem Moment in den Mitnahmeeingriff, in dem der Nadelschutz die Freigabeposition erreicht. In einer zweiten Ausführungsform gelangen das Eingriffselement und das Eingriffsgegenelement in den Mitnahmeeingriff, bevor der Nadelschutz die Freigabeposition erreicht hat. In derartigen Ausführungen ist die Nadelschutzvorrichtung "frühauslösend", da der Mitnahmeeingriff längs des vom Nadelschutz beim Einstechen der Injektionsnadel zurücklegbaren Wegs früher als in der ersten Ausführungsform hergestellt wird. Bevorzugterweise gelangen das Eingriffselement und das Eingriffsgegenelement bereits in einer ersten Hälfte des Wegs in den Mitnahmeeingriff, den der Nadelschutz aus einer distalen Ausgangsposition bis zum Erreichen der Freigabeposition zurücklegt, noch bevorzugter gelangen das Eingriffselement und das Eingriffsgegenelement bereits in einem ersten Drittel dieses Wegstücks in den Mitnahmeeingriff. Die distale Ausgangsposition nimmt der Nadelschutz in einem Ausgangszustand vor Gebrauch der Nadelschutzvorrichtung ein. Der Mitnahmeeingriff ist ein Rasteingriff, in dem das Eingriffselement das Eingriffsgegenelement in Bezug auf die distale Richtung hintergreift. Das Sicherungsglied erstreckt sich bei in Freigabeposition befindlichem Nadelschutz vorteilhafterweise in die distale Richtung so weit, dass die Injektionsnadel das Sicherungsglied nur mit einem Injektionsabschnitt überragt, der bei der Injektion in das Gewebe, vorzugsweise in oder durch die menschliche Haut, ragt.

Durch die Anordnung eines Sicherungsglieds radial zwischen dem Nadelschutz und der Injektionsnadel wird der Nadelschutz in radialer Richtung versteift. Mit dem Sicherungsglied wird zwar ein weiteres Element eingeführt, die Form des Nadelschutzes oder der Blockiereinrichtung einerseits und insbesondere des Nadelhalters andererseits kann jedoch vereinfacht werden. Wegen der radial geschachtelten Anordnung können die für die Blockierung des Nadelschutzes und den Mitnahmeeingriff erforderlichen Formelemente einfach gestaltet werden.

Eine Aussteifung kann dadurch erzielt werden, dass das Sicherungsglied den Nadelschutz bei der Bewegung in die Freigabeposition in einem Gleitkontakt führt. Besonders vorteilhaft ist es, wenn die Blockiereinrichtung und das Sicherungsglied zwischen sich einen Ringspalt bilden, in dem der Nadelschutz sowohl an einer Mantelinnenfläche als auch an einer Mantelaußenfläche je im Gleitkontakt geführt wird. In solch einem Ringspalt ist vorteilhafterweise auch eine mechanische Rückstellfeder für den Nadelschutz angeordnet, wobei der Ringspalt vorteilhafterweise auch die Rückstellfeder axial führt. Ein Ringraum kann alternativ auch als gasdichte Ringkammer gebildet sein, deren Volumen durch die Bewegung des Nadelschutzes verringert oder vergrößert wird, um durch einen auf diese Weise erzeugten Über- oder Unterdruck die Elastizitätskraft für die Rückstellung des Nadelschutzes in die distale Richtung zu erzeugen.

Das Sicherungsglied ist in einer vorteilhaften Weiterbildung so geformt, dass es zumindest in einem axialen Abschnitt, bevorzugt an einem distalen Ende, vorzugsweise über den größten Teil seiner Länge, die Injektionsnadel anliegend oder in einem nur geringen Abstand umgibt. Vorzugsweise weist es zumindest an seinem distalen Ende, vorzugsweise über seine gesamte Länge eine dünne Bohrung auf, die als Passbohrung für die Injektionsnadel geformt ist, so dass das Sicherungsglied bei seiner Bewegung in die distale Richtung mit der Injektionsnadel in einem Gleitkontakt ist. Die Bohrung weist vorteilhafterweise gegenüber dem Außendurchmesser der Injektionsnadel nur das für die Passung erforderliche Übermaß auf. Auf diese Weise steift das Sicherungsglied die in bevorzugten Ausführungen nur dünne Injektionsnadel von beispielsweise 29 bis 32G oder dünner gegen Biegen aus.

Vorzugsweise ragt das Sicherungsglied bei in der Schutzposition befindlichem Nadelschutz über die Nadelspitze in die distale Richtung vor. Hierauf resultiert ein optimaler Schutz vor Stichverletzungen. Ferner kann das den Schutz gewährleistende, in die distale Richtung gemessene Übermaß des Sicherungsglieds und letztlich auch des Nadelschutzes hierdurch minimiert werden, wodurch wiederum die Länge der Nadelschutzvorrichtung insgesamt reduziert werden können.

In bevorzugten Ausführungen dient das Eingriffselement des Nadelschutzes nicht nur der Mitnahme des Sicherungsglieds, sondern in Doppelfunktion auch der Blockierung des Nadelschutzes in der Schutzposition. In derartigen Ausführungen stößt das Eingriffselement in der Schutzposition des Nadelschutzes in die proximale Richtung gegen einen Blockieranschlag der Blockiereinrichtung, d.h. eine Bewegung des Nadelschutzes in die proximale Richtung wird durch Anschlagkontakt des Eingriffselements verhindert. Für eine derartige Blockierung in der Schutzposition ist eine radiale Aussteifung des Nadelschutzes mittels des Sicherungsglieds besonders vorteilhaft, indem das Sicherungsglied dem Eingriffselement eine radiale Stütze bietet, um in der Schutzposition eine Bewegung des Eingriffselements nach radial innen, d.h. auf die Injektionsnadel zu, zu blockieren. Das Eingriffselement kann somit nicht versehentlich oder bewusst aus dem Blockiereingriff mit dem Blockieranschlag bewegt werden.

Das Eingriffselement wird bei der Bewegung des Nadelschutzes in die Freigabeposition vorzugsweise gegen eine rückstellende Elastizitätskraft nach radial außen und durch die Elastizitätskraft in den Mitnahmeeingriff bewegt. In dem Mitnahmeeingriff nimmt es vorzugsweise eine Position ein, die weiter radial außen gelegen ist als die Position, die es vor der Injektion im Ausgangszustand eingenommen hat. Die beiden Positionen sind so gelegen, dass das Eingriffselement in den bevorzugten Ausführungen, in denen es in der Schutzposition die Blockierung des Nadelschutzes bewirkt, an dem genannten Blockieranschlag der Blockiereinrichtung vorbei in den Mitnahmeeingriff bewegt werden kann, nach der Bewegung des Nadelschutzes in die Schutzposition jedoch sicher axial auf Anschlag gegen den Blockieranschlag der Blockiereinrichtung gelangt.

Der Blockieranschlag der Blockiereinrichtung ist in einer bevorzugten Ausführung gegen eine rückstellende Elastizitätskraft nach radial außen bewegbar. Insbesondere kann eine elastisch biegbare Zunge den Blockieranschlag bilden. Vorteilhaft ist es, wenn die Zunge der Blockiereinrichtung in die distale Richtung vorragt und dementsprechend ein freies distales Ende aufweist. Das freie distale Ende bildet vorzugsweise den Blockieranschlag.

Das Sicherungsglied bildet in bevorzugten Ausführungen eine Führungsbahn für das Eingriffselement des Nadelschutzes. Bei der Bewegung des Nadelschutzes in die proximale Richtung, d.h. in Richtung auf die Freigabeposition, wird das Eingriffselement längs der Führungsbahn gegen eine rückstellende Elastizitätskraft bevorzugterweise nach radial außen bewegt, bis es in den Mitnahmeeingriff schnappt. Die Führungsbahn weist über ihren axialen Verlauf gesehen eine Neigung zu der Achse proximal-distal auf. Die Neigung sollte kontinuierlich, d.h. stetig differenzierbar sein. Sie kann beispielsweise konstant sein oder in die proximale Richtung abnehmen oder, bevorzugter, in die proximale Richtung zunehmen, mit der stärksten Zunahme vorzugsweise in der zweiten proximalen Hälfte oder im letzten proximalen Drittel ihres Verlaufs. Falls wie bevorzugt das Eingriffselement als elastisch biegbare Zunge geformt ist, sollte diese Zunge in die proximale Richtung vorragen und dementsprechend ein freies proximales Ende aufweisen.

Vorteilhafte Merkmale werden auch in den Unteransprüchen und deren Kombinationen beschrieben.

Nachfolgend wird die Erfindung anhand eines Ausführungsbeispiels erläutert. An dem Ausführungsbeispiel offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: ein erstes Ausführungsbeispiel einer Nadelschutzvorrichtung in einer perspektivischen Sicht,
- Figur 2: die Nadelschutzvorrichtung des ersten Ausführungsbeispiels in einem Ausgangszustand,
- Figur 3: die Nadelschutzvorrichtung des ersten Ausführungsbeispiels mit in Freigabeposition befindlichem Nadelschutz,
- Figur 4: die Nadelschutzvorrichtung des ersten Ausführungsbeispiels mit in Schutzposition befindlichem Nadelschutz,
- Figur 5: ein zweites Ausführungsbeispiel einer Nadelschutzvorrichtung in Explosionsdarstellung,
- Figur 6: die Nadelschutzvorrichtung des zweiten Ausführungsbeispiels in einem Ausgangszustand,
- Figur 7: ein drittes Ausführungsbeispiel einer Nadelschutzvorrichtung in Explosionsdarstellung,
- Figur 8: die Nadelschutzvorrichtung des dritten Ausführungsbeispiels im zusammengebauten Zustand in einer perspektivischen Sicht,
- Figur 9: die Nadelschutzvorrichtung des dritten Ausführungsbeispiels in einem Ausgangszustand,
- Figur 10: die in dem Ausgangszustand befindliche Nadelschutzvorrichtung des dritten Ausführungsbeispiels in einem anderen Längsschnitt als Figur 9,
- Figur 11: die Nadelschutzvorrichtung des dritten Ausführungsbeispiels mit in einer Auslöseposition befindlichem Nadelschutz,
- Figur 12: die Nadelschutzvorrichtung des dritten Ausführungsbeispiels mit in Freigabeposition befindlichem Nadelschutz und
- Figur 13: die Nadelschutzvorrichtung des dritten Ausführungsbeispiels mit in Schutzposition befindlichem Nadelschutz.

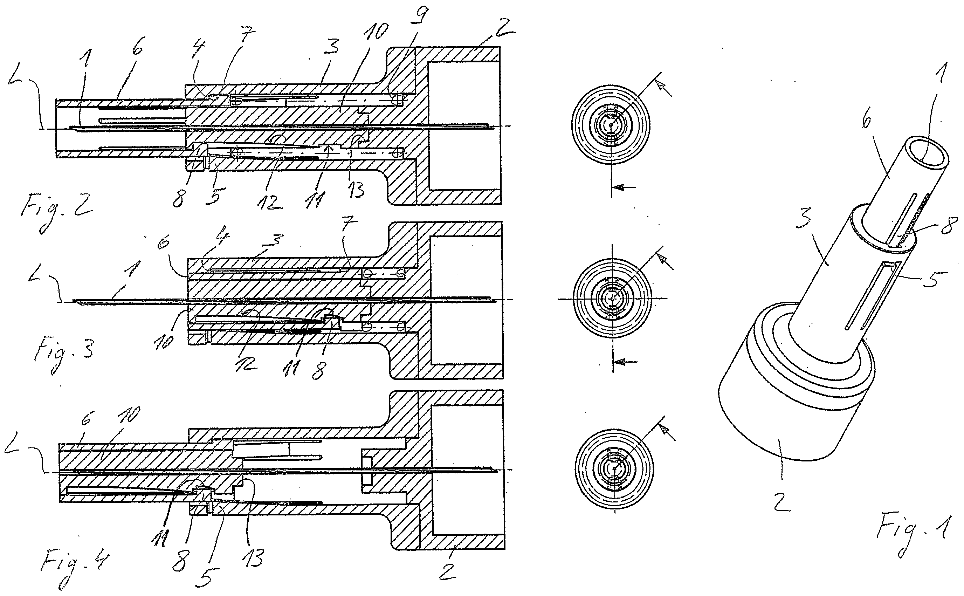

Figur 1 zeigt in perspektivischer Darstellung eine Nadelschutzvorrichtung, die auf ein distales Ende eines Injektionsgeräts aufsteckbar ist. Bei dem Injektionsgerät handelt es sich vorzugsweise um eines, dass die Einstellung einer Dosis eines injizierbaren Produkts, beispielsweise Insulin, und die wiederholte Abgabe der eingestellten Dosis erlaubt. Vorzugsweise kann die Dosis pro Injektion auch erneut eingestellt werden. Das Injektionsgerät ist bevorzugterweise der Art nach ein Injektionspen, wie er in der Selbstverabreichung von Medikamenten, beispielsweise in der Diabetestherapie, üblich ist. Das Injektionsgerät ist somit für mehrere Injektionen, vorzugsweise für eine längere Gebrauchsdauer, ausgelegt. Die Nadelschutzvorrichtung, die eine Injektionsnadel 1 für das Injektionsgerät umfasst, ist hingegen für nur eine einzige Injektion, d.h. nur für einen einmaligen Gebrauch, bestimmt. Sie wird für den Gebrauch an dem distalen Ende des Injektionsgeräts befestigt, beispielsweise aufgeschraubt oder wie im Ausführungsbeispiel aufgesteckt und nach der Injektion abgenommen und entsorgt.

Die Nadelschutzvorrichtung umfasst wie bereits erwähnt die Injektionsnadel 1, einen Nadelhalter 2 und zwei hülsenförmige Strukturen, nämlich eine äußere Hülse 3 und eine innere Hülse 6, die ein Nadelschutzteleskop bilden. Die innere Hülse 6 ist relativ zu der äußeren Hülse 3 und insbesondere der Injektionsnadel 1 axial beweglich. Figur 1 zeigt die Nadelschutzvorrichtung in einem Ausgangszustand, in dem die innere Hülse 6 relativ zu der äußeren Hülse 3 eine distale Position einnimmt, in der sie die Injektionsnadel 1 bis über deren Nadelspitze verdeckt und so einen Sichtschutz bildet. Die innere Hülse 6 wird im Folgenden deshalb als Nadelschutz 6 bezeichnet. Die äußere Hülse 3 dient der Blockierung des Nadelschutzes 6 nach der Injektion und wird im Folgenden deshalb als Blockiereinrichtung 3 bezeichnet.

Figur 2 zeigt die Nadelschutzvorrichtung ebenfalls in dem Ausgangszustand. Neben dem Längsschnitt ist auch ein Querschnitt im Bereich der Blockiereinrichtung 3 dargestellt. In der Querschnittdarstellung ist die Lage des Längsschnitts angegeben.

Der Nadelhalter 2 weist im wesentlichen die Form eines Topfs auf mit einem Boden, von dem in die proximale Richtung umlaufend eine Wandung abragt, die einen Befestigungsabschnitt bildet, mit dem der Nadelhalter 2 an dem Injektionsgerät befestigt wird. Von dem Boden ragt in die distale Richtung im zentralen Bereich ein Halteabschnitt für die Injektionsnadel 1 ab. Die Längsachse L der Injektionsnadel 1 fällt mit der Achse proximal-distal zusammen. Falls das Injektionsgerät wie bevorzugt das zu injizierende Produkt mittels eines Hubkolbens fördert, fällt die axiale Richtung vorteilhafterweise mit der Vortriebsrichtung des Kolbens zusammen. In dem Halteabschnitt ist die Injektionsnadel 1 axial fest mit dem Nadelhalter 2 verbunden. Die Injektionsnadel 1 durchragt den Halteabschnitt des Nadelhalters 2. Sie ragt in die proximale Richtung in einen von der Wandung umgebenen Raum des Befestigungsabschnitts, steht hinter einem proximalen Rand der Wandung jedoch zurück. Dieser Raum wird von einer Folie, die an dem proximalen Ende an der Wandung des Nadelhalters 2 befestigt ist, steril verschlossen. Bei der Befestigung der Nadelschutzvorrichtung an dem Injektionsgerät wird die Folie zerstört, und die Injektionsnadel dringt mit ihrem proximalen spitzen Ende durch eine Membran, die ein distales Ende eines mit dem Produkt gefüllten Behälters verschließt. Auf diese Weise wird bei der Befestigung an dem Injektionsgerät gleichzeitig auch die Injektionsnadel 1 fluidisch mit dem Produktbehälter verbunden.

Zu ihrem distalen Ende hin weist die Injektionsnadel 1 einen Injektionsabschnitt mit der Nadelspitze auf. Die Länge der Injektionsnadel ist für eine subkutane Injektion bemessen. Entsprechend dringt der Injektionsabschnitt in die Haut und zum Teil durch die Haut bis zu einer subkutanen Injektionsstelle. Der Injektionsabschnitt erstreckt sich von der freien Nadelspitze in die proximale Richtung bis auf die axiale Höhe des distalen Endes der Blockiereinrichtung 3. Der Nadelschutz 6 nimmt in dem Ausgangszustand eine distalste Position ein, die durch das Zusammenwirken eines Anschlags 4 der Blockiereinrichtung 3 mit einem Gegenanschlag 7 des Nadelschutzes 6 vorgegeben ist. Den Anschlag 4 bildet eine von der Blockiereinrichtung 3 nach radial innen vorstehende Schulter, und den Gegenanschlag 7 bildet eine von dem Nadelschutz 6 nach radial außen vorstehende Schulter.

Die Nadelschutzvorrichtung umfasst ferner ein Rückstellglied 9, im Ausfübrungsbeispiel eine mechanische Druckfeder. Das Rückstellglied 9 übt auf den Nadelschutz 6 eine in die distale Richtung wirkende Elastizitätskraft aus, mit der es den Gegenanschlag 7 gegen den Anschlag 4 drückt. Das Rückstellglied 9 ist axial an der Blockiereinrichtung 6 und dem Nadelhalter 2 abgestützt.

Die Nadelschutzvorrichtung umfasst ferner ein Sicherungsglied 10 für den Nadelschutz 6. Das Sicherungsglied 10 verlängert den Halteabschnitt des Nadelhalters 2 in die distale Richtung. Es weist eine zylindrische, im Ausführungsbeispiel kreiszylindrische Mantelaußenfläche auf, die einer zylindrischen, parallel beabstandeten, im Ausführungsbeispiel kreiszylindrischen Mantelinnenfläche der Blockiereinrichtung 3 zugewandt gegenüberliegt. Die Mantelaußenfläche des Sicherungsglieds 10 und die Mantelinnenfläche der Blockiereinrichtung 3 begrenzen radial einen axial erstreckten Ringspalt, in den der Nadelschutz 6 einfahren kann. In dem Ringraum ist auch das Rückstellglied 9 angeordnet. Die den Ringraum zwischen sich begrenzenden Mantelflächen führen den Nadelschutz 6 bei seiner Bewegung in einem Gleitkontakt und führen im Ausführungsbeispiel auch das Rückstellglied 9 bei dessen Ein- und Ausfedern.

Die von der Mantelaußenfläche des Sicherungsglieds 10 für den Nadelschutz 6 gebildete Gleitführung wird von zwei Führungsbahnen 12 unterbrochen. Die Führungsbahnen 12 sind einander über die Injektionsnadel 1 diametral gegenüberliegend an der Mantelaußenfläche des Sicherungsglieds 10 geformt. Sie sind axial lang gestreckt und weisen zu der Axialen je eine Neigung auf. Im Längsschnitt der Figur 2 ist aufgrund der Lage des Schnitts nur eine der beiden Führungsbahnen 12 erkennbar. Die andere Führungsbahn 12 entspricht der im Schnitt erkennbaren. Die Führungsbahn 12 weist in Bezug auf die Axiale eine Neigung nach radial außen auf, die in die proximale Richtung zunimmt.

Der Nadelschutz 6 weist zwei Eingriffselemente 8 auf, von denen je eines mit einer der Führungsbahnen 12 zusammenwirkt. Im Schnitt der Figur 2 ist nur eines der Eingriffselemente 8 erkennbar. Das andere Eingriffselement 8 entspricht dem dargestellten der Form nach und auch in seinem Zusammenwirken mit der anderen Führungsbahn 12. Das Eingriffselement 8 ist als biegeelastische Zunge geformt, die sich von einem Wurzelbereich im Mantel des Nadelschutzes 6 in die proximale Richtung erstreckt und an ihrem proximalen Ende eine nach radial innen vorspringende Schulter aufweist. Durch die Schulter wird an dem proximalen Ende des Eingriffselements 8 ein nach innen vorragender Nocken erhalten. Dieser Nocken ist mit der Führungsbahn 12 in einem Gleitkontakt. Bei einer Bewegung des Nadelschutzes 6 in die proximale Richtung gleitet der Nocken auf der Führungsbahn 12, so dass das Eingriffselement 8 aufgrund des geneigten Verlaufs der Führungsbahn 12 allmählich immer stärker elastisch nach außen gebogen wird. Am proximalen Ende der Führungsbahn 12 ist an der Mantelaußenfläche des Sicherungsglieds 10 eine Vertiefung geformt, die ein Eingriffsgegenelement 11 bildet. Der Übergang zwischen der Führungsbahn 12 und dem Eingriffsgegenelement 11 ist abrupt, d.h. die Führungsbahn 12 fällt an ihrem Ende steil in die das Eingriffsgegenelement 11 bildende Vertiefung ab. Das gleiche gilt auf der gegenüberliegenden Seite für die in Figur 2 nicht dargestellte Führungsbahn 12, d.h. dort ist ein ebensolches Eingriffsgegenelement 11 geformt. Zudem sorgt auch das in der dargestellten distalen Ausgangsposition bereits an der Führungsbahn 12 anliegende Eingriffselement 8 für die Rückhaltung des Sicherungsglieds.

Das Sicherungsglied 10 ist mit dem Nadelhalter 2 zwar ausreichend fest verbunden, so dass es die in Figur 2 dargestellte Position nicht aufgrund von Beschleunigungen verlässt, beispielsweise bei Vibrationen oder Stößen, wie sie beim Transport oder der Handhabung üblicherweise auftreten können. Andererseits ist die Verbindung jedoch lösbar. Die Verbindung wird durch Reibschluss zwischen dem Nadelhalter 2, nämlich dessen Halteabschnitt, und dem Sicherungsglied 10 bewirkt. Das Sicherungsglied 10 sitzt an seinem proximalen Ende mit festem Reibsitz in einer an dem distalen Ende des Halteabschnitts des Nadelhalters 2 geformten Aufnahme. Bei den reibschlüssig zusammenwirkenden Sitzflächen handelt es sich um die Mantelinnenfläche der Aufnahme des Nadelhalters 2 und eine dementsprechend als Mantelaußenfläche gebildete Reibschlussfläche 13 des Sicherungsglieds 10. Die Reibschlussfläche 13 ist die Mantelaußenfläche eines Sockels, der an der proximalen Stirnseite des Sicherungsglieds 10 in die proximale Richtung abragt. Die Reibschlussfläche 13 verjüngt sich vorzugsweise konisch in die proximale Richtung, und die Reibschlussfläche des Nadelhalters 2 ist angepasst geformt.

Nach einem Lösen der Reibschlussverbindung ist das Sicherungsglied 10 relativ zu der Injektionsnadel 1 in die distale Richtung gleitbeweglich. Die Injektionsnadel 1 durchragt das Sicherungsglied 10, das hierfür eine zentrale Bohrung aufweist, die in Passung zu dem Außendurchmesser der Injektionsnadel 1 gefertigt ist. Das Sicherungsglied 10 umschließt daher die Injektionsnadel 1 eng anliegend und stützt sie auf diese Weise bis zu seinem distalen Ende gegen Biegeverformungen. Allerdings sollte bei einer Gleitbewegung des Sicherungsglieds 10 relativ zu der Injektionsnadel 1 nur eine möglichst geringe Reibung auftreten. Das Sicherungsglied 10 ist daher vorteilhafterweise aus einem Material gefertigt, vorzugsweise einem Kunststoffmaterial, mit niedrigem Reibkoeffizienten.

Das Sicherungsglied 10 insgesamt weist die Form eines schlanken Bolzens auf. Es steift den Nadelschutz 6 und insbesondere dessen Eingriffselemente 8 in Bezug auf die radiale Richtung aus.

Die Blockiereinrichtung 3 ist in einem proximalen Endabschnitt fest mit dem Nadelhalter 2 verbunden. Soweit es um die Funktion der Blockiereinrichtung 3 in Bezug auf den Schutz vor der Injektionsnadel 1 geht, könnte die Blockiereinrichtung 3 mit dem Nadelhalter 2 in einem Stück geformt sein. Für den Zusammenbau der Nadelschutzvorrichtung ist jedoch eine Fertigung separat von dem Nadelhalter 2 vorteilhaft.

Eine wesentliche Funktion der Blockiereinrichtung 3 ist die Herstellung eines Blockiereingriffs mit dem Nadelschutz 6, um diesen in einer distalen Schutzposition gegen eine Bewegung in die proximale Richtung zu blockieren. In der Schutzposition nimmt der Nadelschutz 6 in Bezug auf die Achse L die gleiche Position wie in dem in Figur 2 dargestellten Ausgangszustand ein. Allerdings ist der Nadelschutz 6 in der Schutzposition an einer Bewegung in die proximale Richtung gehindert, nämlich durch die Blockiereinrichtung 3. Zur Erfüllung der Blockierfunktion weist die Blockiereinrichtung 3 zwei Blockierelemente 5 auf. Die Blockierelemente 5 sind je als eine biegeelastische Zunge geformt. Die Blockierelemente 5 ragen in dem Hülsenmantel der Blockiereinrichtung 3 je von einem proximalen Wurzelbereich in die distale Richtung vorsteht. Die Blockierelemente 5 weisen somit je ein freies distales Ende auf. Die distalen Stirnflächen der Blockierelemente 5 bilden Anschlagflächen für die in der Schutzposition des Nadelschutzes 6 axial zugewandt gegenüberliegenden proximalen Stirnflächen der Eingriffselemente 8. Die Blockierelemente 5 bilden so je einen Blockieranschlag und die Eingriffselemente 8 je einen Blockiergegenanschlag für die Blockierung des Nadelschutzes 6 in der Schutzposition. In der Schutzposition umgibt der Nadelschutz den Injektionsabschnitt der Injektionsnadel 1 bis über deren Nadelspitze hinaus.

In dem Ausgangszustand, wie Figur 2 ihn zeigt, sind der Blockieranschlag des Blockierelements 5 und der Blockiergegenanschlag des Eingriffselements 8 außer Eingriff. Das Eingriffselement 8 liegt zumindest im Wesentlichen spannungsfrei an dem distalen Ende der Führungsbahn 12 an. In dieser Position kann es bei dem Einfahren des Nadelschutzes 6 radial innen an den Blockierelement 5 ungehindert vorbeibewegt werden. Gleiches gilt für das in Umfangsrichtung um 180⁰ versetzt angeordnete Paar aus Blockierelement 5 und Eingriffselement 8. Obgleich die Nadelschutzvorrichtung bevorzugt mehrere Paare der zusammenwirkenden Elemente 5 und 8 umfasst und dies vorteilhafterweise in symmetrischer Anordnung bezüglich der Achse L, genügt zur Blockierung grundsätzlich bereits ein einziges Elementenpaar 5, 8.

Im Folgenden wird der Ablauf einer Injektion anhand der Figuren 2 bis 4 geschildert:
Nachdem der Verwender die Nadelschutzvorrichtung wie eingangs beschrieben an dem Injektionsgerät befestigt hat, wird die Injektionsnadel 1 entlüftet. Anschließend wählt der Verwender die zu injizierende Dosis. Nach Auswahl der Dosis setzt er das Injektionsgerät mit der distalen Stirnfläche des Nadelschutzes 6 an der gewünschten Einstechstelle auf die Haut und drückt das Injektionsgerät gegen die Haut. Durch diesen Druck in die distale Richtung fährt der Nadelschutz 6 axial gegen die Elastizitätskraft des Rückstellglieds 9 in den Ringspalt zwischen der Blockiereinrichtung 3 und dem Sicherungsglied 10 ein. Der Nadelschutz 6 ist relativ zu der Blockiereinrichtung 3 axial verdrehgesichert geführt. Im distal ersten Abschnitt der Bewegung gleiten die Eingriffselemente 8 längs der jeweils zugeordneten Führungsbahn 12. Die Führungsbahnen 12 sind in ihren distalen Endabschnitten entweder axial gerade oder nur sehr schwach nach außen geneigt, so dass die Eingriffselemente 8 ohne weiteres radial innen an dem jeweils zugeordneten Blockierelement 5 vorbeifahren können. Im weiteren Verlauf der Bewegung werden die Eingriffselemente 8 längs der sich radial aufweitenden Führungsbahnen 12 elastisch nach außen gebogen. Die Blockiereinrichtung 3 weitet sich im Umfangsbereich ihrer Blockierelemente 5 in die proximale Richtung soweit auf, dass sich die Eingriffselemente 8 nahezu ungehindert, im Idealfall ohne die Blockierelemente 5 zu verformen, nach außen biegen können. An den proximalen Enden der Führungsbahnen 12 bewegen sich die Eingriffselemente 8 über die dort die Eingriffsgegenelemente 11 distal begrenzenden Kanten und unter ihrer eigenen rückstellenderi Elastizitätskraft nach innen; sie schnappen so in die Eingriffsgegenelemente 11 ein. Die Eingriffselemente 8 und Eingriffsgegenelemente 11 sind nun miteinander paarweise in einem Mitnahmeeingriff verhakt.

Das Blockierelement 5 ist an seiner der Führungsbahn 12 zugewandten Innenseite wie bereits erwähnt vorzugsweise so geformt, das zwischen der Innenseite und der Führungsbahn 12 über den gesamten Hub des Nadelschutzes 6 ein ausreichend großer Abstand verbleibt, damit das Eingriffselement 8 keinen oder zumindest keinen das Blockierelement 5 nach außen gerichteten Druck auf das Blockierelement 5 ausübt. Das Eingriffsgegenelement 11 und die Führungsbahn 12 sind ferner so geformt, dass das Eingriffselement 8 im Mitnahmeeingriff im Vergleich zu seiner distalen Ausgangsposition nach außen abgebogen ist, im Mitnahmeeingriff also einen größeren Abstand von der zentralen Längsachse L als in der distalen Ausgangsposition aufweist. Die Eingriffselemente 8 liegen somit beim Einfahren in die Freigabeposition tiefer in der jeweils zugeordneten Führungsbahn 12 als bei dem Ausfahren. Im Mitnahmeeingriff weist eine dem Blockierelement 5 zugewandte Außenseite des Eingriffselements 8 von der zentralen Längsachse L einen größeren Abstand auf als das Blockierelement 5 im distalen Bereich an seiner Innenseite.

Figur 3 zeigt die Nadelschutzvorrichtung mit den im Mitnahmeeingriff befindlichen Eingriffselementen 8 und Eingriffsgegenelementen 11. Eine weitere Bewegung des Nadelschutzes 6 in die proximale Richtung wird durch den Mitnahmeeingriff verhindert. Darüber hinaus ist der Nadelschutz 6 über seine gesamte Länge in die axiale Überlappung mit der Blockiereinrichtung 3 eingefahren. In diesem voll eingefahrenen Zustand liegen die distalen Stirnenden der Blockiereinrichtung 3, des Nadelschutzes 6 und des Sicherungsglieds 10 auf der gleichen axialen Höhe. Die Injektionsnadel 1 ragt über das gemeinsame Stirnende mit ihrem gesamten Injektionsabschnitt vor, d.h. die Injektionsnadel 1 ist bis in die gewünschte, längs der Injektionsnadel 1 gemessene Eindringtiefe durch die Haut in das darunter liegende subkutane Gewebe eingedrungen, und es kann die eingestellte Dosis verabreicht werden. Während der Verabreichung muss lediglich der für die Überwindung der rückstellenden Elastizitätskraft des Rückstellglieds 9 erforderliche Druck ausgeübt werden. Nachdem die eingestellte Dosis verabreicht wurde, entfernt der Verwender das Injektionsgerät axial in die proximale Richtung von der Einstichstelle, so dass der Druck von dem Nadelschutz 6 genommen wird. Der Nadelschutz 6 fährt daher unter der Wirkung des Rückstellglieds 9 axial in die distale Richtung wieder aus und zurück in seine distalste Position, die durch das Zusammenwirken des Anschlags 4 und des Gegenanschlags 7 bestimmt wird.

Figur 4 zeigt die Nadelschutzvorrichtung nach der Injektion mit dem in der distalsten Position befindlichen Nadelschutz 6. Am Ende der Bewegung des Nadelschutzes 6 in die distale Richtung verriegelt die Nadelschutzvorrichtung mittels der zusammenwirkenden Eingriffselemente 8 und Blockierelemente 5 automatisch, so dass die distalste Position nun die blockierte Schutzposition des Nadelschutzes 6 ist. Bei der Bewegung in die distale Richtung nimmt der Nadelschutz 6 aufgrund des Mitnahmeeingriffs bei 5, 8 das Sicherungsglied 10 in die proximale Richtung mit. Da die Eingriffselemente 8 auch im Mitnahmeeingriff noch nach radial außen gebogen sind und die Blockierelemente 5 an ihren den Eingriffselementen 8 zugewandten Innenflächen in die distale Richtung nach innen geneigt sind gelangen die Eingriffselemente 8 im Verlaufe der Ausfahrbewegung in Gleitkontakt mit der Innenfläche des jeweils zugeordneten Blockierelements 5, so dass die Blockierelemente 5 elastisch nach außen gebogen werden. Am Ende der Bewegung in die distale Richtung kommen die Eingriffselemente 8 mit ihren proximalen Stirnflächen vor die Blockierelemente 5 zu liegen, so dass die Blockierelemente 5 aufgrund ihrer eigenen rückstellenden Elastizitätskraft nach innen schnappen und je einen Blockieranschlag für das jeweils zugeordnete Eingriffselement 8 bilden. Der Nadelschutz 6 ist auf diese Weise in der Schutzposition blockiert.

Das Sicherungsglied 10 stützt die Eingriffselemente 8 nach radial innen ab, so dass diese nicht aus dem mit den Blockierelementen 5 gebildeten Blockiereingriff bewegt werden können. Die Blockierelemente 5, Eingriffselemente 8 und Führungsbahnen 12 sind vorteilhafterweise so geformt, dass die Blockierelemente 5 nur bei der Bewegung des Nadelschutzes 6 in die distale Richtung in einem relevanten Ausmaß elastisch beansprucht werden. Die sich bei dem Einfahren nach außen biegenden Eingriffselemente 8 sollten demgegenüber allenfalls eine deutlich kleinere Biegung der Blockierelemente 5 verursachen. Bevorzugt werden die Blockierelemente 5 beim Einfahren überhaupt nicht belastet. Dies wird im Ausführungsbeispiel durch den im Längsschnitt geneigten Verlauf der Innenflächen der Blockierelemente 5 und der Führungsbahnen 12 und schlanke Eingriffselemente 8 erreicht.

Die Eingriffsgegenelemente 11 sind in Bezug auf die axiale Richtung so angeordnet, dass sie in der Schutzposition des Nadelschutzes 6 axial von der Blockiereinrichtung 3 überlappt werden, so dass die Blockiereinrichtung 3 Manipulationen der Eingriffselemente 8, durch die die Eingriffselemente 8 aus dem Mitnahmeeingriff bewegt werden könnten, verhindert.

Wie Figur 4 des Weiteren zeigt, bietet das Sicherungsglied 10 mit seiner zur Injektionsnadel 1 auf Passung gefertigten Bohrung einen besonders sicheren Schutz vor Stichverletzungen. Die Nadelspitze steht in der Bohrung ein kleines Stück weit hinter der Mündung der Bohrung zurück. Ein versehentlicher Kontakt mit der Injektionsnadel 1 ist somit in der Schutzposition nicht mehr möglich.

Der Verwender nimmt nach der Injektion die Nadelschutzvorrichtung von dem Injektionsgerät ab und führt sie der Entsorgung zu. Für eine erneute Injektion wird eine neue, im Ausgangszustand der Figur 2 befindliche Nadelschutzvorrichtung an dem Injektionsgerät befestigt.

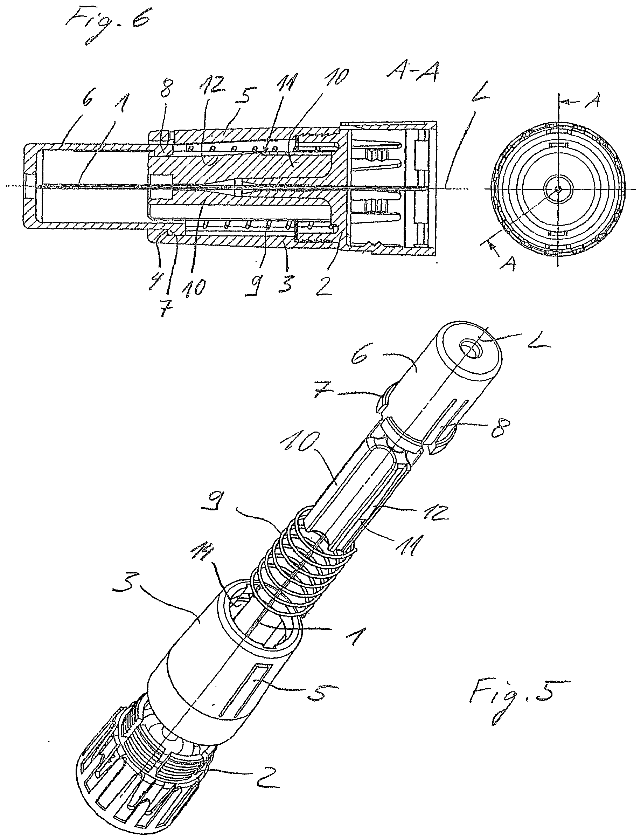

Die Figuren 5 und 6 zeigen eine Nadelschutzvorrichtung nach einem zweiten Ausführungsbeispiel. Die Nadelschutzvorrichtung unterscheidet sich von der

Nadelschutzvorrichtung des ersten Ausführungsbeispiels im Wesentlichen nur in Bezug auf das Sicherungsglied 10, das im zweiten Ausführungsbeispiel im Querschnitt gesehen mehrere axial erstreckte Rippen aufweist, die aus einem zentralen Bereich abragen. Im Ausführungsbeispiel weist das Sicherungsglied 10 vier solcher Rippen auf, die in Umfangsrichtung um 90° zueinander versetzt sind, so dass im Querschnitt im Großen und Ganzen eine Kreuzform erhalten wird. Die Rippen ragen jeweils bis an die Mantelinnenfläche des Nadelschutzes 6. Am äußeren Umfang von zwei der Rippen ist je eine der Führungsbahnen 12 und je am proximalen Ende der Führungsbahn 12 ein Eingriffsgegenelement 11 geformt. Das Zusammenwirken der Eingriffselemente 8 mit den Führungsbahnen 12 und den Eingriffsgegenelementen 11 entspricht dem ersten Ausführungsbeispiel, d. h. der Mitnahmeeingriff wird erst bei Erreichen der Freigabeposition hergestellt. Die Nadelschutzvorrichtung des ersten und des zweiten Ausführungsbeispiels sind in diesem Sinne spätauslösend. In Figur 5 sind auch die Eingriffsmittel für die verdrehgesicherte Führung des Nadelschutzes 6 relativ zu der Blockiereinrichtung 3 erkennbar. Die Führung wird durch den Eingriff der Gegenanschläge 7 mit Axialführungen 14 gebildet, die an der Mantelinnenfläche der Blockiereinrichtung 3 geformt sind.

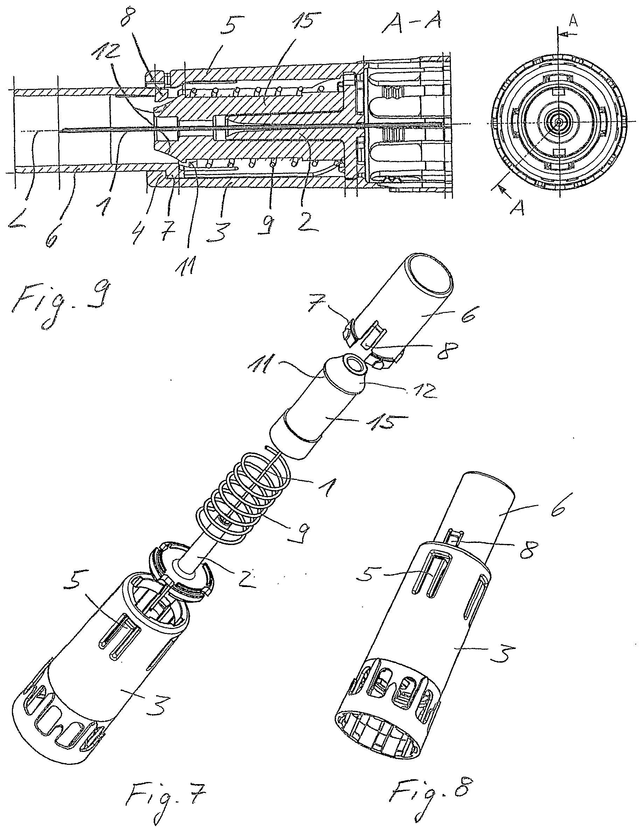

Figur 7 zeigt eine Nadelschutzvorrichtung eines dritten Ausführungsbeispiels in einer Explosionsdarstellung, in der die Komponenten der Nadelschutzvorrichtung längs der zentralen Längsachse L einzeln dargestellt sind. Die Nadelschutzvorrichtung des dritten Ausführungsbeispiels ist "frühauslösend" und weist für die Frühauslösung ein hinsichtlich der Führungsbahn 12 und des Eingriffsgegenelements 11 modifiziertes Sicherungsglied 15 auf. Ein weiterer, allerdings weniger gravierender Unterschied besteht hinsichtlich des Nadelhalters 2 und der Blockiereinrichtung 3. Im dritten Ausführungsbeispiel ist der Nadelhalter 2 in die hülsenförmige Blockiereinrichtung 3 eingesetzt, und die Blockiereinrichtung 3 bildet den Befestigungsabschnitt für die Befestigung der Nadelschutzvorrichtung an einem Injektionsgerät. Der Nadelhalter 2 ist wie in Figur 9 erkennbar in die Blockiereinrichtung 3 eingesetzt und im eingesetzten Zustand mit der Blockiereinrichtung 3 axial und um die Längsachse L rotatorisch nicht beweglich verbunden.

Figur 8 zeigt die Nadelschutzvorrichtung nach dem Zusammenbau der in Figur 7 einzeln dargestellten Komponenten mit dem in der distalen Ausgangsposition befindlichen Nadelschutz 6.

Figur 9 zeigt die Nadelschutzvorrichtung des dritten Ausführungsbeispiels in einem Querschnitt und einem in dem Querschnitt eingezeichneten Längsschnitt A-A.

Zum Zwecke der Frühauslösung ist die das Eingriffsgegenelement 11 bildende Schulter, die in dem Mitnahmeeingriff von dem Eingriffselement 8 hintergriffen wird, in einem distalen Endabschnitt des Sicherungsglieds 15 geformt. Die distal vor dem Eingriffsgegenelement 11 angeordnete Führungsbahn 12 ist entsprechend der Anordnung des Eingriffsgegenelements 11 im Vergleich zu den Führungsbahnen 12 der anderen Ausführungsbeispiele deutlich kürzer. Sie weist auch eine stärkere Neigung zur Längsachse L auf als die Führungsbahnen 12 der anderen Ausführungsbeispiele. Im dritten Ausführungsbeispiel bildet eine um die Längsachse L umlaufende äußere Umfangsfläche des Sicherungsglieds 15 die Führungsbahn 12 für jedes der Eingriffselemente 8, wobei der Nadelschutz 6 auch im dritten Ausführungsbeispiel zwei Eingriffselemente 8 aufweist. Das Sicherungsglied 12 fällt am distalen Ende der Führungsbahn in einer steilen, ebenfalls um die Längsachse L umlaufenden Schulter nach radial innen ab. Die Schulter bildet für jedes der Eingriffselemente 8 das Eingriffsgegenelement 11.

Nachfolgend wird die Nadelschutzvorrichtung bezüglich der für die Frühauslösung bestimmenden Komponenten und deren Funktion beschrieben. Im Übrigen wird auf die Ausführungen zu den beiden anderen Ausführungsbeispielen verwiesen. Insbesondere werden für die mit den beiden anderen Ausführungsbeispielen funktionsgleichen Komponenten des dritten Ausführungsbeispiels die Bezugszeichen der beiden anderen Ausführungsbeispiele verwendet.

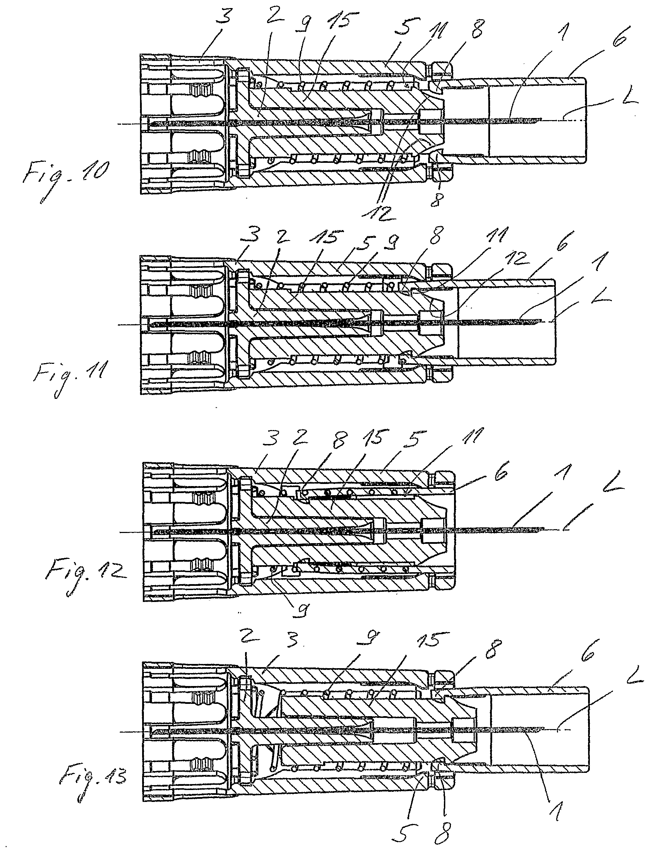

Die Funktionsweise der Nadelschutzvorrichtung des dritten Ausführungsbeispiels wird nachfolgend anhand der Figuren 10 bis 13 erläutert, wobei im Wesentlichen nur auf die Frühauslösung eingegangen wird:
Figur 10 zeigt die Nadelschutzvorrichtung in einem Ausgangszustand vor Gebrauch, in dem der Nadelschutz 6 die distale Ausgangsposition einnimmt. In der Ausgangsposition wird der Nadelschutz 6 durch die zusammenwirkenden Anschläge 4 und 7 (Figur 9) an der Blockiereinrichtung 3 wie in den anderen Ausführungsbeispielen gehalten. Die beiden Eingriffselemente 8 liegen distal unmittelbar vor der Führungsbahn 12 und sind entspannt. Wird auf den Nadelschutz 6 in die proximale Richtung ein axialer Druck ausgeübt, bewegt sich der Nadelschutz 6 relativ zu der Blockiereinrichtung 3 und dem in seiner proximalen Ausgangsposition befindlichen Sicherungsglied 15 in die proximale Richtung. Gleich zu Beginn der Bewegung gelangen die Eingriffselemente 8 in Führungskontakt mit der Führungsbahn 12 und biegen sich entsprechend der Neigung der Führungsbahn 12 nach radial außen ab. Die Eingriffselemente 8 fahren längs der Führungsbahn 12 in den Spalt zwischen dem Sicherungsglied 15 und den Blockierelementen 5, wobei sie stetig stärker nach radial außen abgebogen werden, bis das proximale Ende der Führungsbahn 12 erreicht ist und die Eingriffselemente 8 aufgrund ihrer elastischen . Rückstellkraft hinter das Eingriffsgegenelement 11 nach radial innen schnappen, so dass der Mitnahmeeingriff hergestellt ist.

Figur 11 zeigt die Nadelschutzvorrichtung mit dem in der Auslöseposition befindlichen Nadelschutz 6, d. h. mit dem im Mitnahmeeingriff befindlichen Nadelschutz 6. Aufgrund der im Vergleich zu den beiden anderen Ausführungsbeispielen in die distale Richtung weit vorgerückten Position des Eingriffsgegenelements 11 ist der Mitnahmeeingriff bereits hergestellt, bevor die Spitze der Injektionsnadel 1 in die distale Richtung über den Nadelschutz 6 vorsteht. Im Ausführungsbeispiel steht die Nadelspitze sogar noch ein Stück weit hinter dem Nadelschutz 6 zurück. Weiterer Druck auf den Nadelschutz 6 bewirkt, dass der Nadelschutz 6 gegen die Kraft des Rückstellelements 9 weiter in die Blockiereinrichtung 3 einfährt. Die Eingriffselemente 8 fahren bei dieser weiteren Einfahrbewegung über die axial gerade, im Ausführungsbeispiel kreiszylindrische Umfangsfläche, die sich in die proximale Richtung an das Eingriffsgegenelement 11 anschließt.

Figur 12 zeigt die Nadelschutzvorrichtung mit dem in der Freigabeposition befindlichen Nadelschutz 6. Axialer Druck auf den Nadelschutz 6 bewirkt, dass der Nadelschutz 6 gegen die Kraft des Rückstellelements 9 in der Freigabeposition verbleibt. Wird der axiale Druck von dem Nadelschutz 6 genommen, fährt der Nadelschutz 6 aufgrund der rückstellenden Kraft des Rückstellelements 9 automatisch wieder in die distale Richtung vor, bis die Eingriffselemente 8 in Anschlagkontakt mit dem Eingriffsgegenelement 11 gelangen. Der Abstand, den das Eingriffsgegenelement 11 in der Ausgangsposition (Figur 10) von der Anlagefläche des Nadelhalters 2 aufweist, ist so bemessen, dass das Sicherungsglied 15 in dem nun wieder erreichten Mitnahmeeingriff der Eingriffselemente 8 und des Eingriffsgegenelements 11 von dem Nadelschutz 6 in die distale Richtung mitgenommen wird. Im Verlaufe der gemeinsamen Bewegung des Nadelschutzes 6 und des Sicherungsglieds 15 bewegen sich die Eingriffselemente 8 in die distale Richtung vor die Blockierelemente 5 der Blockiereinrichtung 3. Wie in den beiden anderen Ausführungsbeispielen befinden sich die Eingriffselemente 8, d. h. deren im Eingriff befindliche Enden, radial weiter außen als in der Ausgangsposition des Nadelschutzes. Bei der Ausfahrbewegung werden die Blockierelemente 5 daher nach radial auswärts abgebogen und schnappen aufgrund ihrer eigenen elastischen Rückstellkraft wieder nach radial einwärts vor, sobald sie von den Eingriffselementen 8 passiert worden sind.

Figur 13 zeigt die Nadelschutzvorrichtung des dritten Ausführungsbeispiels in der Schutzposition, in der die Blockierelemente 5 proximal vor die im Mitnahmeeingriff befindlichen Eingriffselemente 8 geschnappt sind und Blockieranschläge für die Eingriffselemente 8 und somit für den Nadelschutz 6 bilden.

### Bezugszeichen:

- 1: Injektionsnadel
- 2: Nadelhalter
- 3: Blockiereinrichtung, äußere Hülse
- 4: Anschlag
- 5: Blockierelement
- 6: Nadelschutz, innere Hülse
- 7: Gegenanschlag
- 8: Eingriffselement
- 9: Rückstellglied
- 10: Sicherungsglied
- 11: Eingriffsgegenelement
- 12: Führungsbahn
- 13: Reibschlussfläche
- 14: Axialführung
- 15: Sicherungsglied

- L: Längsachse

## Patentansprüche

1. Nadelschutzvorrichtung für ein oder an einem Injektionsgerät, die Nadelschutzvorrichtung umfassend:
a) eine Injektionsnadel (1),
b) einen an dem Injektionsgerät befestigten oder befestigbaren Nadelhalter (2), von dem die Injektionsnadel (1) in eine distale Richtung vorragt,
c) eine Blockiereinrichtung (3), die von dem Nadelhalter (2) vorragt und radial von der Injektionsnadel beabstandet ist,
d) einen Nadelschutz (6)
e) und ein Rückstellglied (9), das auf den Nadelschutz (6) eine in die distale Richtung wirkende Elastizitätskraft ausübt, wobei
f) der Nadelschutz (6) radial zwischen der Injektionsnadel (1) und der Blockiereinrichtung (3) in die proximale Richtung bis in eine die Injektionsnadel (1) freigebende Freigabeposition und aus der Freigabeposition in die distale Richtung bis in eine Schutzposition bewegbar ist, in der er die Injektionsnadel (1) einschliesslich einer Nadelspitze umgibt,
g) und der mittels der Blockiereinrichtung (3) in der Schutzposition gegen eine erneute Bewegung in die proximale Richtung blockierbar ist, wobei
h) radial zwischen der Injektionsnadel (1) und dem Nadelschutz (6) ein Sicherungsglied (10; 15) relativ zu der Injektionsnadel (1) aus einer proximalen Position in die distale Richtung bewegbar angeordnet ist,
i) der Nadelschutz (6) ein Eingriffselement (8) und das Sicherungsglied (5) ein Eingriffsgegenelement (11) aufweisen, die miteinander in einen Mitnahmeeingriff gelangen, wenn der Nadelschutz (6) in die Freigabeposition bewegt wird,
j) und dass der Nadelschutz (6) in dem Mitnahmeeingriff das Sicherungsglied (10; 15) bei der Bewegung in die Schutzposition mitnimmt,
**dadurch gekennzeichnet, dass**
k) der Mitnahmeeingriff ein Rasteingriff ist, in dem das Eingriffselement (8) das Eingriffsgegenelement (11) in Bezug auf die distale Richtung hintergreift.

2. Nadelschutzvorrichtung nach dem vorhergehenden Anspruch, **dadurch gekennzeichnet, dass** das Sicherungsglied (10; 15) den Nadelschutz (6) in einem Gleitkontakt führt.

3. Nadelschutzvorrichtung nach einem der vorhergehenden Ansprüche, **dadurch gekennzeichnet, dass** das Sicherungsglied (10; 15) an dem Nadelhalter (2) mittels Reibschluss (13) lösbar gehalten ist.

4. Nadelschutzvorrichtung nach einem der vorhergehenden Ansprüche, **dadurch gekennzeichnet, dass** das Sicherungsglied (10; 15) zumindest an seinem distalen Ende die Injektionsnadel (1) in einem geringen radialen Abstand umgibt oder vorzugsweise in einem Gleitkontakt mit der Injektionsnadel (1) ist.

5. Nadelschutzvorrichtung nach dem vorhergehenden Anspruch, **dadurch gekennzeichnet, dass** sich das Sicherungsglied (10) in der proximalen Position bis zu einem distalen Ende des Nadelschutzes (6) erstreckt, wenn der Nadelschutz die Freigabeposition einnimmt.

6. Nadelschutzvorrichtung nach einem der vorhergehenden Ansprüche, **dadurch gekennzeichnet, dass** das Sicherungsglied (10; 15) in dem Mitnahmeeingriff ebenfalls bis in eine Schutzposition bewegbar ist, in der es die Injektionsadel (2) einschliesslich der Nadelspitze umgibt.

7. Nadelschutzvorrichtung nach einem der vorhergehenden Ansprüche, **dadurch gekennzeichnet, dass** das Eingriffselement (8) in der Schutzposition des Nadelschutzes (6) in die proximale Richtung gegen einen Blockieranschlag (5) der Blockiereinrichtung (3) stösst.

8. Nadelschutzvorrichtung nach dem vorhergehenden Anspruch, **dadurch gekennzeichnet, dass** der Blockieranschlag (5) gegen eine Elastizitätskraft nach radial aussen beweglich ist.

9. Nadelschutzvorrichtung nach einem der zwei vorhergehenden Ansprüche, **dadurch gekennzeichnet, dass** eine in Längsrichtung (L) der Injektionsnadel (1) erstreckte biegeelastische erste Zunge den Blockieranschlag (5) und eine in Längsrichtung der Injektionsnadel (1) erstreckte biegeelastische zweite Zunge das Eingriffselement (8) bilden.

10. Nadelschutzvorrichtung nach dem vorhergehenden Anspruch, **dadurch gekennzeichnet, dass** die erste Zunge ein freies distales Ende und die zweite Zunge ein freies proximales Ende aufweist.

11. Nadelschutzvorrichtung nach einem der vorhergehenden Ansprüche, **dadurch gekennzeichnet, dass** das Eingriffselement (8) gegen eine Elastizitätskraft nach radial aussen beweglich ist.

12. Nadelschutzvorrichtung nach einem der vorhergehenden Ansprüche, **dadurch gekennzeichnet, dass** das Sicherungsglied (10; 15) das Eingriffselement (8) nach radial innen stützt, um in der Schutzposition eine Bewegung des Eingriffselements (8) nach radial innen zu blockieren.

13. Nadelschutzvorrichtung nach einem der vorhergehenden Ansprüche, **dadurch gekennzeichnet, dass** das Sicherungsglied (10; 15) eine Führungsbahn (12) für das Eingriffselement (8) aufweist, die in proximaler Richtung mit einer Neigung nach radial aussen verläuft, um das Eingriffselement (8) bei der Bewegung in die Freigabeposition nach aussen zu lenken.

14. Nadelschutzvorrichtung nach dem vorhergehenden Anspruch, **dadurch gekennzeichnet, dass** die Führungsbahn (12) an einem proximalen Ende nach radial innen abfällt, um das Eingriffsgegenelement (11) zu bilden.

15. Nadelschutzvorrichtung nach einem der zwei vorhergehenden Ansprüche, **dadurch gekennzeichnet, dass** die Führungsbahn (12) so geformt ist, vorzugweise in Bezug auf eine Längsachse (L) der Injektionsnadel (1) solche eine Neigung aufweist, dass das Eingriffselement (8) und das Eingriffsgegenelement (11) in den Mitnahmeeingriff gelangen, bevor der Nadelschutz (6) die Freigabeposition erreicht hat, vorzugsweise in einer ersten Hälfte des vom Nadelschutz (6) aus einer durch Anschlag (4, 7) bestimmten distalen Ausgangsposition in die Freigabeposition zurückzulegenden Wegs.

## Claims

1. A needle guard device for or on an injection device, the needle guard device including:
a) an injection needle (1),
b) a needle holder (2) which is or can be fixed to the injection device and from which the injection needle (1) projects in a distal direction,
c) a blocking device (3) which projects from the needle holder (2) and is radially spaced from the injection needle,
d) a needle guard (6), and
e) a return member (9) which exerts on the needle guard (6) an elasticity force operative in the distal direction, wherein
f) the needle guard (6) is moveable radially between the injection needle (1) and the blocking device (3) in the proximal direction into a release position freeing the injection needle (1) and out of the release position in the distal direction into a guard position in which it surrounds the injection needle (1) including a needle point,
g) and which can be blocked by means of the blocking device (3) in the guard position against renewed movement in the proximal direction, wherein
h) a securing member (10; 15) is arranged between the injection needle (1) and the needle guard (6) moveably relative to the injection needle (1) from a proximal position in the distal direction,
i) the needle guard (6) has an engagement element (8) and the securing member (5) has a counterpart engagement element (11) which comes into entrainment engagement with each other when the needle guard (6) is moved into the release position,
j) and the needle guard (6) in the entrainment engagement entrains the securing member (10; 15) upon the movement into the guard position,
**characterised in that**
k) the entrainment engagement is a latching engagement in which the engagement element (8) engages behind the counterpart engagement element (11) in relation to the distal direction.

2. A needle guard device according to the preceding claim **characterised in that** the securing member (10; 15) guides the needle guard (6) in sliding contact.

3. A needle guard device according to one of the preceding claims **characterised in that** the securing member (10; 15) is releasably held to the needle holder (2) by means of frictional locking.

4. A needle guard device according to one of the preceding claims **characterised in that** the securing member (10; 15) at least at its distal end surrounds the injection needle (1) at a small radial spacing or is preferably in sliding contact with the injection needle (1).

5. A needle guard device according to the preceding claim **characterised in that** the securing member (10) in the proximal position extends to a distal end of the needle guard (6) when the needle guard assumes the release position.

6. A needle guard device according to one of the preceding claims **characterised in that** the securing member (10;15) is also moveable in entrainment engagement into a guard position in which it surrounds the injection needle (2) including the needle point.

7. A needle guard device according to one of the preceding claims **characterised in that** the engagement element (8) in the guard position of the needle guard (6) butts in the proximal direction against a blocking abutment (5) of the blocking device (3).

8. A needle guard device according to the preceding claim **characterised in that** the blocking abutment (5) is moveable radially outwardly against an elasticity force.

9. A needle guard device according to one of the two preceding claims **characterised in that** a flexurally elastic first tongue extending in the longitudinal direction (L) of the injection needle (1) forms the blocking abutment (5) and a flexurally elastic second tongue extending in the longitudinal direction of the injection needle (1) forms the engagement element (8).

10. A needle guard device according to the preceding claim **characterised in that** the first tongue has a free distal end and the second tongue has a free proximal end.

11. A needle guard device according to one of the preceding claims **characterised in that** the engagement element (8) is moveable radially outwardly against an elasticity force.

12. A needle guard device according to one of the preceding claims **characterised in that** the securing member (10; 15) supports the engagement element (8) radially inwardly in order to block a movement of the engagement element (8) radially inwardly in the guard position.

13. A needle guard device according to one of the preceding claims **characterised in that** the securing member (10; 15) has a guide path (12) for the engagement element (8) which extends with an inclination radially outwardly in the proximal direction in order to deflect the engagement element (8) outwardly in the movement into the release position.

14. A needle guard device according to the preceding claim **characterised in that** the guide path (12) falls away radially inwardly at a proximal end to form the counterpart engagement element (11).

15. A needle guard device according to one of the two preceding claims **characterised in that** the guide path (12) is so formed and preferably in relation to a longitudinal axis (L) of the injection needle (1) has such an inclination that the engagement element (8) and the counterpart engagement element (11) come into entrainment engagement before the needle guard (6) has reached the release position, preferably in a first half of the distance to be covered by the needle guard (6) from a distal starting position determined by abutments (4, 7) into the release position.

## Revendications

1. Dispositif de protection d'aiguille pour un ou au niveau d'un appareil d'injection, le dispositif de protection d'aiguille comprenant :
a) une aiguille d'injection (1),
b) un porte-aiguille (2) fixé ou pouvant être fixé au niveau de l'appareil d'injection, duquel l'aiguille d'injection (1) fait saillie dans une direction distale,
c) un dispositif de blocage (3), qui fait saillie du porte-aiguille (2) et est espacé radialement de l'aiguille d'injection,
d) une protection d'aiguille (6)
e) et un organe de rappel (9), qui exerce une force d'élasticité agissant dans la direction distale sur la protection d'aiguille (6), dans lequel
f) la protection d'aiguille (6) est déplaçable radialement entre l'aiguille d'injection (1) et le dispositif de blocage (3) dans la direction proximale jusque dans une position de libération libérant l'aiguille d'injection (1) et depuis la position de libération dans la direction distale jusque dans une position de protection, dans laquelle elle entoure l'aiguille d'injection (1) y compris une pointe d'aiguille,
g) et qui peut être bloquée au moyen du dispositif de blocage (3) dans la position de protection contre un nouveau déplacement dans la direction proximale, dans lequel
h) un organe de sécurisation (10 ; 15) est agencé radialement entre l'aiguille d'injection (1) et la protection d'aiguille (6) de manière déplaçable par rapport à l'aiguille d'injection (1) depuis une position proximale dans la direction distale,
i) la protection d'aiguille (6) présente un élément de mise en prise (8) et l'organe de sécurisation (5) présente un contre-élément de mise en prise (11), qui parviennent l'un avec l'autre en une mise en prise d'entraînement, lorsque la protection d'aiguille (6) est déplacée dans la position de libération,
j) et la protection d'aiguille (6) entraîne dans la mise en prise d'entraînement l'organe de sécurisation (10 ; 15) lors du déplacement dans la position de protection,
**caractérisé en ce que**
k) la mise en prise d'entraînement est une mise en prise par enclenchement, dans laquelle l'élément de mise en prise (8) vient en prise derrière le contre-élément de mise en prise (11) par rapport à la direction distale.

2. Dispositif de protection d'aiguille selon la revendication précédente, **caractérisé en ce que** l'organe de sécurisation (10 ; 15) guide la protection d'aiguille (6) en contact glissant.

3. Dispositif de protection d'aiguille selon l'une quelconque des revendications précédentes, **caractérisé en ce que** l'organe de sécurisation (10 ; 15) est maintenu de manière amovible au niveau du porte-aiguille (2) par liaison par frottement (13).

4. Dispositif de protection d'aiguille selon l'une quelconque des revendications précédentes, **caractérisé en ce que** l'organe de sécurisation (10 ; 15) entoure au moins au niveau son extrémité distale l'aiguille d'injection (1) à une faible distance radiale ou est de préférence en contact glissant avec l'aiguille d'injection (1).

5. Dispositif de protection d'aiguille selon la revendication précédente, **caractérisé en ce que** l'organe de sécurisation (10) s'étend dans la position proximale jusqu'à une extrémité distale de la protection d'aiguille (6), lorsque la protection d'aiguille prend la position de libération.

6. Dispositif de protection d'aiguille selon l'une quelconque des revendications précédentes, **caractérisé en ce que** l'organe de sécurisation (10 ; 15) est déplaçable dans la mise en prise d'entraînement également jusqu'à une position de protection, dans laquelle il entoure l'aiguille d'injection (2) y compris la pointe d'aiguille.

7. Dispositif de protection d'aiguille selon l'une quelconque des revendications précédentes, **caractérisé en ce que** dans la position de protection de la protection d'aiguille (6) dans la direction proximale l'élément de mise en prise (8) heurte une butée de blocage (5) du dispositif de blocage (3).

8. Dispositif de protection d'aiguille selon la revendication précédente, **caractérisé en ce que** la butée de blocage (5) est mobile radialement vers l'extérieur contre une force d'élasticité.

9. Dispositif de protection d'aiguille selon l'une des deux revendications précédentes, **caractérisé en ce qu'**une première languette élastique en flexion s'étendant dans la direction longitudinale (L) de l'aiguille d'injection (1) forme la butée de blocage (5) et une seconde languette élastique en flexion s'étendant dans la direction longitudinale de l'aiguille d'injection (1) forme l'élément de mise en prise (8).

10. Dispositif de protection d'aiguille selon la revendication précédente, **caractérisé en ce que** la première languette présente une extrémité distale libre et la seconde languette présente une extrémité proximale libre.

11. Dispositif de protection d'aiguille selon l'une quelconque des revendications précédentes, **caractérisé en ce que** l'élément de mise en prise (8) est mobile radialement vers l'extérieur contre une force d'élasticité.

12. Dispositif de protection d'aiguille selon l'une quelconque des revendications précédentes, **caractérisé en ce que** l'organe de sécurisation (10 ; 15) soutient l'élément de mise en prise (8) radialement vers l'intérieur pour bloquer dans la position de protection un déplacement de l'élément de mise en prise (8) radialement vers l'intérieur.

13. Dispositif de protection d'aiguille selon l'une quelconque des revendications précédentes, **caractérisé en ce que** l'organe de sécurisation (10 ; 15) présente une voie de guidage (12) pour l'élément de mise en prise (8), qui s'étend dans la direction proximale avec une inclinaison radialement vers l'extérieur pour diriger l'élément de mise en prise (8) vers l'extérieur lors du déplacement dans la position de libération.

14. Dispositif de protection d'aiguille selon la revendication précédente, **caractérisé en ce que** la voie de guidage (12) est inclinée radialement vers l'intérieur au niveau d'une extrémité proximale pour former le contre-élément de mise en prise (11).

15. Dispositif de protection d'aiguille selon l'une des deux revendications précédentes, **caractérisé en ce que** la voie de guidage (12) est formée de sorte à présenter de préférence par rapport à un axe longitudinal (L) de l'aiguille d'injection (1) une inclinaison telle que l'élément de mise en prise (8) et le contre-élément de mise en prise (11) parviennent dans la mise en prise d'entraînement, avant que la protection d'aiguille (6) n'ait atteint la position de libération, de préférence dans une première moitié du chemin à parcourir par la protection d'aiguille (6) depuis une position de départ distale déterminée par butée (4, 7) à la position de libération.
